# EUROPÄISCHE PATENTANMELDUNG

(11) **EP 1 132 043 A2**
(43) Veröffentlichungstag der Anmeldung: **12.09.2001**
(21) Anmeldenummer: 01114093.6
(22) Anmeldetag: 02.05.1997
(51) Int. Cl.: A61B 3/024, A61B 3/06

(54) **Gerät zur Prüfung des Gesichtsfeldes des menschlichen Auges**

(30) Priorität: 24.06.1996 DE 19625199
(62) Teilanmeldung aus: 97107274.9
(71) Anmelder: Oculus Optikgeräte GmbH, 35582 Wetzlar (DE)
(72) Erfinder: Köst, Gert, Dipl.-Biologe, 30171 Hannover (DE)
(74) Vertreter: Missling, Arne, Dipl.-Ing.

(57) **Zusammenfassung**

Geräte zur Prüfung des Gesichtsfeldes des menschlichen Auges mit einem schalenförmigen Schirm, auf dessen Grund eine oder mehrere Testmarken projiziert werden, sollten sowohl für die kinetische wie auch statische Perimetrie einsetzbar sein und Testmarken gleichmäßiger Größe und Helligkeit über dem gesamten Prüfbereich anbieten können. Zu diesem Zweck besteht der Schirm aus einem transparenten Material, wobei die Testmarken von der Rückseite her auf den Schirm etwa senkrecht zur Flächennormalen projiziert werden. Die Testmarke ist dabei flächig über die Rückseite des Schirmes verfahrbar angeordnet, wobei jede Testmarke in einem äquidistanten Abstand zum Schirm verfahrbar ist.

## Beschreibung

Die Erfindung betrifft ein Gerät zur Prüfung des Gesichtsfeldes des menschlichen Auges mit einem schalenförmigen Schirm, auf dessen Grund eine oder mehrere Testmarken projiziert werden nach dem Oberbegriff des Anspruches 1. Ein derartiges Gerät ist aus der CH-PS 264 664 bekannt.

In der augenärztlichen Praxis spielt die Messung des Gesichtsfeldes neben der Druckmessung eine wesentliche Rolle. Bekannt ist es, zur Messung des Gesichtsfeldes das Perimeter von Goldmann einzusetzen, das es gestattet, eine kinetische-quantitative wie auch eine statisch-quantitative Untersuchung durchzuführen. Diese kinetisch-quantitative Perimetrie arbeitet mit einer beweglichen Testmarke bestimmter Leuchtdichte, mit der man die Stellen gleicher Unterschiedsempfindlichkeit senkrecht auf die zu erwartenden Gesichtsfeldgrenzen bzw. Isopteren aufsucht, was durch die freie Beweglichkeit der Testmarke in jeder Richtung gewährleistet wird. Bei der statisch-quantitativen Perimetrie wird dem Probanden eine feststehende Testmarke mit bestimmter Leuchtdichte angeboten, die an einer beliebigen Stelle im Gesichtsfeld als überschwellig erkannt wird. Beide Untersuchungsmethoden werden in der Regel nebeneinander durchgeführt, so dass ein Gerät vorteilhaft ist, mit dem beide Untersuchungsmethoden durchgeführt werden können.

Das Goldmann-Perimeter ist ein schalenförmiges Projektionsperimeter mit direkter Registrierung der Testmarkenposition. Die gleichmäßige und konstante Ausleuchtung der Innenseite der Schale soll bei jeder Untersuchung stets den gleichen Adaptionszustand des Auges gewährleisten. Die Testmarke, mit welcher man die Reizschwelle des Lichtsinnes in den zentralen und peripheren Gesichtsfeldpartien bestimmt, wird auf den Grund der Schale von der Schalenöffnung her projiziert. Da jedoch der Probandenkopf genau auf das Zentrum der Schale fixiert ist, ist es zwangsnotwendig, dass das Projektionssystem exzentrisch angeordnet ist. Durch diese exzentrische Anordnung ergeben sich unterschiedlich starke Verzeichnungen der Testmarke in Abhängigkeit der jeweils gewählten Testmarkenposition. Hierdurch ändert sich nicht nur die Form der Testmarke, sondern auch deren Helligkeitsverteilung, so dass hierdurch Fehlerquellen auftreten können.

Der Erfindung liegt die Aufgabe zugrunde, ein Gerät der eingangs genannten Art so auszubilden, dass dieses zum einen für die statische wie auch für die kinetische Perimetrie geeignet ist, das es gestattet, Testmarken gleichmäßiger Größe und gleichmäßiger Helligkeit im gesamten Prüfbereich anzubieten und das darüber hinaus einfach im Aufbau und in der Betriebsweise ist und weiterhin eine Prüfung der Farbtüchtigkeit zuläßt.

Diese Aufgabe wird mit den Merkmalen des Anspruches 1 gelöst.

Ein erfindungsgemäßes Gerät besteht somit aus einem Schirm aus einem transparenten Material, auf dessen Rückseite die Testmarke in etwa senkrecht zur Flächennormale projiziert wird, wobei die Lichtquelle und damit die Testmarke flächig über die Rückseite des Schirmes verfahrbar angeordnet ist. Das Verfahren der Lichtquelle längs der Rückseite des Schirmes erfolgt in etwa einem äquidistanten Abstand zur Oberfläche des Schirmes, so dass die Testmarke stets die gleiche Größe aufweist und durch die Projektion der Testmarke zur Normalen des Schirmes auch überall stets die gleiche Helligkeit hat.

Ein erfindungsgemäßes Gerät hat eine oder mehrere Lichtquellen, die auf einem Schwenkarm angeordnet sind, der entsprechend der Krümmung des Schirmes ausgebildet ist. Dieser Schwenkarm ist um eine Achse drehbar, die senkrecht zur Projektionsfläche des Schirmes verläuft. Die Länge des Schwenkarmes muß mindestens so groß sein, dass dieser bis zum Rand des Schirmes reicht, so dass beim Drehen des Schwenkarmes dieser die gesamte Schirmfläche überstreicht. Der Schwenkarm als solcher ist relativ zu seiner Längsachse verschiebbar, so dass die Lichtquellen mit ihrer Abbildungsoptik zum einen über den halben Umfang des Schirmes verschiebbar sind und durch Drehen des Schwenkarmes um seine Schwenkachse auf jede beliebige Stelle der Oberfläche des Schirmes gelangen können. Der Abstand der Lichtquellen und der Projektionsoptik von der Oberfläche des Schirmes ändert sich bei der Bewegung der Lichtquellen nicht.

Erfindungsgemäß ist der Schwenkarm mittels dreier Führungsrollen an einer an der Drehachse befestigten Montageplatte gehalten, wobei diese Führungsrollen an gegenüberliegenden Seiten des Schwenkarmes angreifen und jeweils einen Abstand zueinander aufweisen. Hierdurch kann zum einen der Schwenkarm relativ zur Achse verschoben werden und behält zum anderen seine Lage relativ zum Schirm stets bei. Als Antriebsmittel dient ein Zahnriemen, der an den Enden des Schwenkarmes befestigt ist und im Bereich eines Antriebsritzels von dem Schwenkarm über Umlenkrollen angehoben wird.

Ein Ausführungsbeispiel der Erfindung ist im folgenden anhand der Zeichnungen näher beschrieben. In diesen zeigen:
- Fig. 1: einen Schnitt durch ein erfindungsgemäß ausgebildetes Gerät,

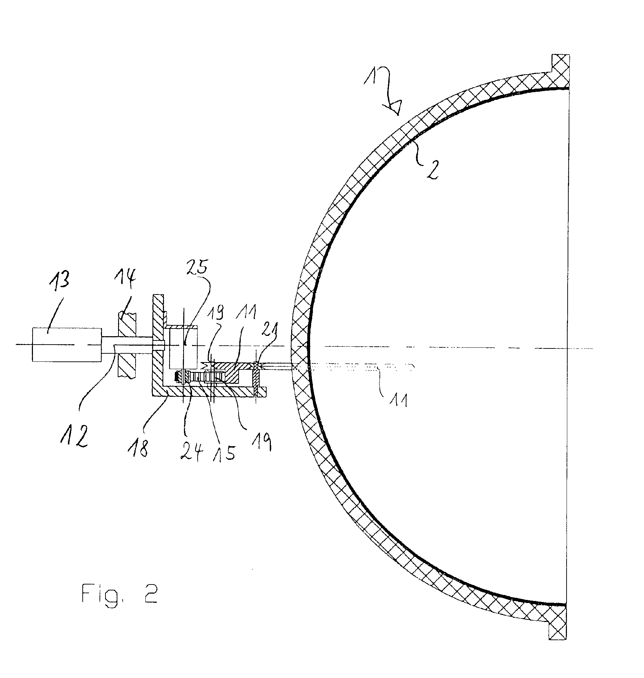

- Fig. 2: einen um 90 ° hierzu verlaufenden Schnitt.

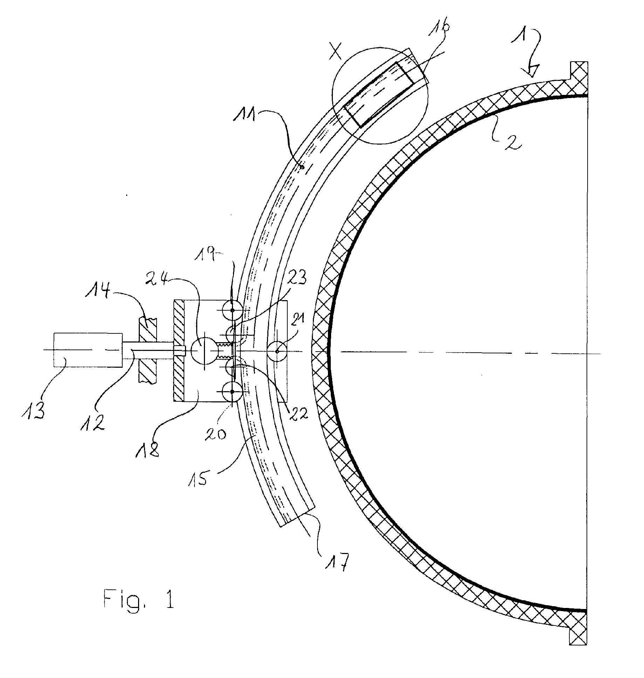

In Fig. 1 ist ein Horizontalschnitt durch ein erfindungsgemäßes Perimeter dargestellt, wobei der Schirm 1 halbkugelförmig ausgebildet ist und auf seiner Innenfläche eine Projektionsfläche 2 aufweist. Der Proband sieht mittig in den Schirm hinein, wobei sein Auge durch eine im Ausführungsbeispiel nicht dargestellte Fixationsmarke fixiert ist. Der Schirm 1 besteht aus einem durchsichtigen oder durchscheinenden Material.

Um eine einfache Verschiebung des Schwenkarmes 11 relativ zu seinen Führungsrollen 19, 20 und 21 zu erhalten, ist an dessen Enden 16, 17 an Zahnriemen 15 befestigt, der über zwei Umlenkrollen 22, 23 von dem Schwenkarm 11 abgehoben wird und über ein Antriebszahnrad 24 geführt wird. Dieses Zahnrad wird von einem Motor 25 angetrieben.

Der Schwenkarm 11 seinerseits ist auf einer Montageplatte 18 befestigt, an der auch die Führungsrollen 19, 20, 21, die Umlenkrollen 22, 23 sowie das Antriebszahnrad 24 befestigt sind. Dies Montageplatte wiederum ist an einer Achse 12 befestigt, die am Rahmen 14 des Gerätes befestigt ist. Diese Achse ist senkrecht zur Projektionsfläche 2 des Schirmes angeordnet, d. h. die Verlängerung der Achse verläuft durch den Mittelpunkt des Schirmes. Wird diese Achse 12 durch den Motor 13 gedreht, so wird die Lichtquelle 9, 10 und damit auch die Testmarke 4 auf einem Kreis bewegt. Durch Verdrehen wie auch durch Verschieben des Schwenkarmes 11 relativ zur Montageplatte 18 und damit zur Achse 12 kann an jeder beliebigen Stelle der Projektionsfläche 2 des Schirmes 1 eine Testmarke erzeugt werden.

## Patentansprüche

1. Gerät zur Prüfung des Gesichtsfeldes des menschlichen Auges mit einem schalenförmigen Schirm, auf dessen Grund eine oder mehrere Testmarken projiziert werden der Schirm (1) aus einem transparenten Material besteht, wobei die Lichtquelle(n) (9, 10) für die Testmarke(n) (4) auf einem der Kontur des Schirmes (1) mit äquidistantem Abstand folgenden und auf dessen Rückseite liegenden Schwenkarm (11) angeordnet ist (sind), der parallel zur Projektionsfläche (2) des Schirmes (1) mittels eines Zahnrades verschiebbar ist, die Testmarke(n) (4) von der Rückseite (3) her auf den Schirm (1) etwa senkrecht zur Flächennormalen projiziert wird (werden) und die Lichtquelle(n) und damit die Testmarke(n) (4) flächig über dessen Rückseite verfahrbar angeordnet ist (sind),
**dadurch gekennzeichnet, dass** der Schwenkarm (11) durch mindestens drei Führungsrollen (19, 20, 21) geführt ist, die auf einer mit der Drehachse (12) verbundenen Montageplatte (18) befestigt sind, und dass auf dem Schwenkarm (11) ein Zahnriemen (15) angeordnet ist, der an dem jeweiligen Ende (16, 17) des Schwenkarmes (11) befestigt ist und über zwei Umlenkrollen (22, 23) und ein Antriebszahnrad (24) geführt ist.
